(19) Europäisches Patentamt
European Patent Office
Office européen des brevets

(11) Veröffentlichungsnummer: **0 240 026 A1**

(12) # EUROPÄISCHE PATENTANMELDUNG

(21) Anmeldenummer: 87104925.0

(22) Anmeldetag: 02.04.87

(51) Int. Cl.³: **C 07 D 401/04**
C 07 D 401/06, C 07 D 401/14
C 07 D 403/04, C 07 D 403/06
C 07 D 403/14, C 07 D 405/04
C 07 D 405/06, C 07 D 405/14
C 07 D 409/04, C 07 D 409/06

(30) Priorität: 04.04.86 DE 3611343

(43) Veröffentlichungstag der Anmeldung: 07.10.87 Patentblatt 87/41

(84) Benannte Vertragsstaaten: AT BE CH DE ES FR GB GR IT LI LU NL SE

(71) Anmelder: **Boehringer Mannheim GmbH**
**Sandhofer Strasse 116**
**D-6800 Mannheim 31(DE)**

(72) Erfinder: **von der Saal, Wolfgang, Dr. rer. nat.**
**Neuer Burgweg 3**
**D-6940 Weinheim(DE)**

(72) Erfinder: **Mertens, Alfred, Dr. rer. nat.**
**Beethovenstrasse 20**
**D-6905 Schriesheim(DE)**

(72) Erfinder: **Friebe, Walter-Gunar, Dr. rer. nat.**
**Sophienstrasse 8**
**D-6800 Mannheim 1(DE)**

(72) Erfinder: **Müller-Beckmann, Bernd, Dr. med. vet.**
**Hochgewanne 46**
**D-6718 Grünstadt(DE)**

(54) **Heterocyclisch substituierte Benzimidazole, Verfahren zu ihrer Herstellung sowie Arzneimittel, die diese Verbindungen enthalten.**

(57) Heterocyclisch substituierte Benzimidazole der allgemeinen Formel I

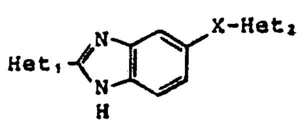

(I)

in welcher

Het₁ einen heterocyclischen Fuenfring mit 1–4 Heteroatomen oder einen heterocyclischen Sechsring mit 1–5 Heteroatomen darstellt, wobei die Heteroatome der vorgenannten Fuenf- und Sechsringe gleich oder verschieden sein koennen und Stickstoff, Sauerstoff oder Schwefelk bedeuten und gegebenenfalls an einem oder mehreren Stickstoffatomen ein Sauerstoffatom tragen koennen, und die vorgenannten Fuenf- und Sechsringe gegebenenfalls durch eine odert mehrere Alkyl-, Alkoxy-, Alkylmercapto-, Hydroxy-, Nitro-, Amino-, Halogen- oder Cyangruppen substituiert sein koennen,

$Het_2$ einen heterocyclischen Sechsring mit 1–5 Heteroatomen darstellt, wobei die Heteroatome gleich oder verschieden sein koennen und Stickstoff, Sauerstoff oder Schwefel bedeuten,
und die Sechsringe gewuenschtenfalls durch eine oder mehrere Alkyl-, Alkoxy-, Alkoxyalkyl-, Alkylmercapto-, Hydroxy-, Hydroxyalkyl-, Amino-, Halogen-, oder Cyangruppen substituiert sein koennen,

X eine Bindung, eine $C_1$–$C_4$-Alkylengruppe oder die Vinylengruppe bedeutet,

deren Tautomere und deren physiologisch vertraegliche Salze anorganischer und organischer Saeuren, Verfahren zu ihrer Herstellung, diese Verbindungen enthaltende Arzneimittel und die Verwendung zur Behandlung von Herz- und Kreislauferkrankungen.

EP 0 240 026 A1

BOEHRINGER MANNHEIM GMBH 2815/00/EP

# Heterocyclisch substituierte Benzimidazole, Verfahren zu ihrer Herstellung sowie Arzneimittel, die diese Verbindungen enthalten

Die vorliegende Erfindung betrifft neue heterocyclisch substituierte Benzimidazole der allgemeinen Formel I

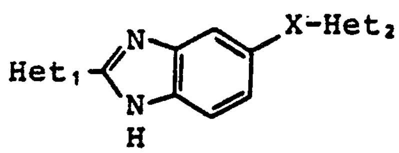

(I)

in welcher

Het₁ einen heterocyclischen Fuenfring mit 1-4 Heteroatomen oder einen heterocyclischen Sechsring mit 1-5 Heteroatomen darstellt, wobei die Heteroatome der vorgenannten Fuenf- und Sechsringe gleich oder verschieden sein koennen und Stickstoff, Sauerstoff oder Schwefel bedeuten und gegebenenfalls an einem oder mehreren Stickstoffatomen ein Sauerstoffatom tragen koennen, und die vorgenannten Fuenf- und Sechsringe gegebenenfalls durch eine oder mehrere Alkyl-, Alkoxy-, Alkylmercapto-, Hydroxy-, Nitro-, Amino-, Halogen- oder Cyangruppen substituiert sein koennen,

Het₂ einen heterocyclischen Sechsring mit 1-5 Heteroatomen darstellt, wobei die Heteroatome gleich oder verschieden sein koennen und Stickstoff, Sauerstoff oder Schwefel bedeuten,

und die Sechsringe gewuenschtenfalls durch eine oder mehrere Alkyl-, Alkoxy-, Alkoxyalkyl-, Alkylmercapto-, Hydroxy-, Hydroxyalkyl-, Amino-, Halogen-, oder Cyangruppen substituiert sein koennen,

X eine Bindung, eine $C_1$-$C_4$-Alkylengruppe oder die Vinylengruppe bedeutet,

deren Tautomere und deren physiologisch vertraeglichen Salze anorganischer und organischer Saeuren und Verfahren zu ihrer Herstellung, sowie diese Verbindungen enthaltende Arzneimittel.

Fuer den Fall, daß Verbindungen der allgemeinen Formel I hergestellt wurden, die ein asymmetrisches Kohlenstoffatom enthalten, sind Gegenstand der Erfindung auch die optisch aktiven Formen und racemischen Gemische dieser Verbindungen.

Diese neuen Verbindungen der vorliegenden Erfindung weisen wertvolle pharmakologische Eigenschaften auf, insbesondere steigern sie die Herzkraft und/oder wirken blutdrucksenkend und/oder verbessern die Mikrozirkulation und beeinflussen die Thrombozytenfunktion.

Verbindungen der allgemeinen Formel I, in der an Stelle von $Het_1$ ein substituierter Phenylring steht, sind schon seit laengerem als positiv inotrop wirkende Stoffe erkannt (vgl.: Europaeische Offenlegungsschrift Nr. 8391).

Ueberraschenderweise konnte gefunden werden, daß die Verbindungen der allgemeinen Formel I eine wesentlich höhere Herzkraft steigernde Wirkung aufweisen als die aus dem Stand der Technik bekannten Verbindungen.

Bedeutet $Het_1$ einen heterocyclischen Fuenfring mit 1-4 Heteroatomen oder einen heterocyclischen Sechsring mit 1-5 Heteroatomen, wobei die Heteroatome der vorgenannten Fuenf- oder Sechsringe gleich oder verschieden sein koennen und Stickstoff, Sauerstoff oder Schwefel bedeuten und gegebenenfalls an einem oder mehreren Stickstoffatomen ein Sauerstoff tragen koennen, so sind in diesem Sinne bevorzugt der Pyrrol-, Furan-, Thiophen-, Pyrazol-, Imidazol-, Thiazol-, Oxazol-, Isoxazol-, Triazol-, Tetrazol-, Thiadiazol-, Oxadiazol-, Pyrazin-, N,N'-Dioxy-pyrazin-, Pyrimidin-, N,N'-Dioxy-pyrimidin-, Pyridazin-, Oxazin-, Thiazin-, Triazin-, Tetrazin-, Pyridyl- und der N-Oxy-pyridylrest.

Alkyl-, Alkoxy-, Alkoxyalkyl- und Alkylmercapto-Substituenten in den heterocyclischen Fuenf- und Sechsringen koennen 1-6, vorzugsweise 1-4 Kohlenstoffatome enthalten. Bevorzugt ist der Methyl-, Ethyl-, Methoxy-, Ethoxy-, Methylmercapto- und Ethylmercaptorest. Unter Halogen ist Fluor, Chlor und Brom, vorzugsweise Chlor zu verstehen.

Bedeutet $Het_2$ einen heterocyclischen Sechsring mit 1-5 gleichen oder verschiedenen Heteroatomen wie Sauerstoff, Schwefel oder Stickstoff, und sind diese Sechsringe gegebenenfalls durch eine oder mehrere Alkyl-, Alkoxy-, Alkoxyalkyl-, Alkylmercapto-, Hydroxy-, Hydroxyalkyl-, Amino-, Halogen- oder Cyangruppen substituiert, so sind in diesem Sinne bevorzugt der 3-Oxo-2,3-dihydro-6-pyridazinyl-, 5-Alkyl-3-oxo-2,3-dihydro-6-pyridazinyl-, 3-Oxo-2,3,4,5-tetrahydro-6-pyridazinyl-, 5-Alkyl-3-oxo-2,3,4,5-tetrahydro-6-pyridazinyl-, 5-Hydroxyalkyl-3-oxo-2,3,4,5-tetrahydro-6-pyridazinyl-, 3-Cyan-6-alkyl-2-oxo-1,2-dihydro-5-pyridinyl-, 6-Alkyl-2-oxo-1,2-dihydro-5-pyridinyl-, 3-Amino-2-oxo-1,2-dihydro-5-pyridinyl-, 3-Amino-6-alkyl-2-oxo-1,2-dihydro-5-pyridinyl-, 3-Hydroxy-6-alkyl-2-oxo-1,2-dihydro-5-pyridinyl-, 6-Oxo-1,6-dihydro-1,2,4-triazin-3-yl-,

6-Oxo-1,4,5,6-tetrahydro-1,2,4-triazin-3-yl-, 5-Oxo-4,5-dihydro-6H-1,3,4-oxadiazin-2-yl-, 5-Oxo-4,5-dihydro-6H-1,3,4-thiadiazin-2-yl-, 3-Oxo-2,3-dihydro-1,2,4-triazin-6-yl-, 3-Oxo-2,3,4,5-tetrahydro-1,2,4-triazin-6-yl-, 2-Oxo-2,3-dihydro-6H-1,3,4-oxadiazin-5-yl-, 2-Oxo-2,3-dihydro-6H-1,3,4-thiadiazin-5-yl-, 5-Alkyl-3-oxo-2,3,4,5-tetrahydro-1,2,4-triazin-6-yl-, 2-Oxo-1,2-dihydro-5-pyrimidinyl-, 4-Alkyl-2-oxo-1,2-dihydro-5-pyrimidinyl-, 2-Oxo-1,2-dihydro-5-pyrazinyl-, 3-Alkyl-2-oxo-1,2-dihydro-5-pyrazinyl-, 6-Alkyl-2-oxo-1,2-dihydro-5-pyrazinylrest.

Fuer X ist der Valenzstrich, die Vinylen- und die Ethylengruppe bevorzugt.

Besonders bevorzugte Verbindung sind Verbindungen der allgemeinen Formel I

in der

$Het_1$ den Pyrrol-, Furan-, Thiophen-, Pyrazol-, Imidazol-, Isoxazol-, Thiazol-, Oxazol-, Triazol-, Tetrazol-, Thiadiazol-, Oxadiazol-, Pyridin-, N-Oxy-pyridin-, Pyrazin-, N,N'-Dioxy-pyrazin-, Pyrimidin-, N,N'-Dioxypyrimidin-, Pyridazin-, Oxazin-, Thiazin-, Triazin- oder Tetrazinrest darstellt, sowie deren Methyl-, Ethyl-, Methoxy-,Hydroxy-, Ethoxy-, Methylmercapto-, Ethylmercapto- und chlorsubstituierten Derivate,

$Het_2$ den 3-Oxo-2,3,4,5-tetrahydro-6-pyridazinyl-, 5-Methyl-3-oxo-2,3,4,5-tetrahydro-6-pyridazinyl-, 5-Hydroxymethyl-3-oxo-2,3,4,5-tetrahydro-6-pyridazinyl-, 3-Cyan-6-methyl-2-oxo-1,2-dihydro-5-pyridinyl-, 3-Oxo-2,3,4,5-tetrahydro-1,2,4-triazin-6-yl, 2-Oxo-4,5-dihydro-6H-1,3,4-oxadiazin-5-yl-, 6-Oxo-1,4,5,6-tetrahydro-1,2,4-triazin-3-yl-,

5-Oxo-4,5-dihydro-6H-1,3,4-oxadiazin-2-yl-, 2-Oxo-1,2-dihydro-5-pyrimidinyl-, 2-Oxo-1,2-dihydro-5-pyrazinyl-,

5-Oxo-4,5-dihydro-6H-1,3,4-thiadiazin-2-yl-, oder den 5-Oxo-4,5-dihydro-6H,1,3,4-thiadiazin-5-yl-rest
und

X einen Valenzstrich, die Vinylengruppe oder die Ethylengruppe bedeutet.

Die Verbindungen der allgemeinen Formel I koennen in an sich bekannter Weise hergestellt werden.

Insbesondere sind die Verfahren fuer die Benzimidazolsynthese beschrieben in

E.S. Schipper und A.R. Day in R.C. Elderfield (Herausgeber) Heterocyclic Compounds, Band 5, S. 194. J. Wiley and Sons, New York 1957.
P.N. Preston in The Chemistry of Heterocyclic Compounds, Band 40, S. 1. J. Wiley and Sons, New York 1981.

Besonders vorteilhaft sind die in den Schemata 1-3 aufgezeigten Synthesewege:

<u>Schema 1</u>

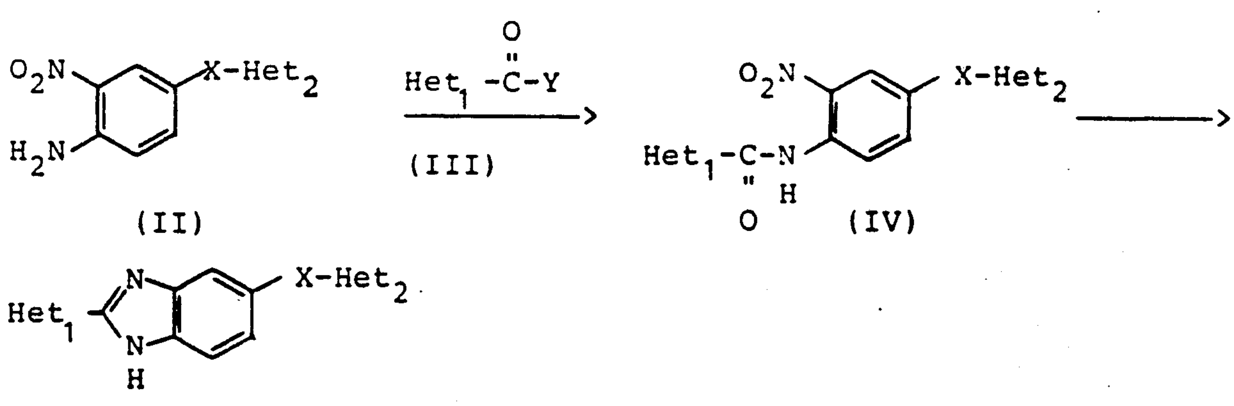

(II)
(III)
(IV)
(I)

Wie aus Schema 1 ersichtlich kann man Verbindungen der allgemeinen Formel II, in der X und $Het_2$ die oben angegebenen Bedeutungen besitzen, durch Verbindungen der allgemeinen Formel III, in der $Het_1$ die oben angegebenen Bedeutungen besitzt und Y die Hydroxygruppe oder einen leicht abspaltbaren Rest darstellt, acylieren. Die dabei entstehenden Amide der allgemeinen Formel IV, in der $Het_1$, $Het_2$ und X die oben genannten Bedeutungen besitzen, lassen sich reduzieren und cyclisieren zu Verbindungen der allgemeinen Formel I.

Die Verbindungen der allgemeinen Formel IV, in der $Het_1$, $Het_2$ und X die oben angegebenen Bedeutungen haben, sind neu und ebenfalls Gegenstand der Erfindung.

Unter den Verbindungen der allgemeinen Formel III versteht man Carbonsaeuren oder deren aktivierten Derivate wie Saeurehalogenide, vorzugsweise Saeurechloride, Carbonsaeureester wie Methyl- und Ethylester, und Anhydride.

Ist die Verbindung der allgemeinen Formel III eine Carbonsaeure, so findet die Umsetzung mit Verbindungen der allgemeinen Formel II in einem inerten Loesungsmittel in Gegenwart eines Wasser bindenden Stoffes bei Temperaturen zwischen 0°C und 100°C, vorzugsweise zwischen Raumtemperatur und 50°C, vorzugsweise in Methylenchlorid in Gegenwart von N,N'-Dicyclohexylcarbodiimid statt. Die Reaktion kann auch in Polyphosphorsaeure ohne weiteren Zusatz oder in Gegenwart von Phosphorpentoxid bei Temperaturen zwischen 50°C und 250°C, vorzugsweise zwischen 100°C und 200°C durchgefuehrt werden.

Ist die Verbindung der allgemeinen Formel III ein Carbonsaeurederivat, so findet die Umsetzung mit Verbindungen der allgemeinen Formel II in inerten Loesungsmitteln, vorzugsweise in Methylenchlorid oder Pyridin statt.

Die Reduktion der Nitrogruppe in Verbindungen der allgemeinen Formel IV erfolgt durch Hydrierung in einem Loesungsmittel wie Wasser, Ethanol, Eisessig, Essigsaeureethylester oder Dimethylformamid zweckmaeßigerweise mit Wasserstoff in Gegenwart eines Hydrierungskatalysators wie Raney-Nickel, Platin oder Palladium auf Kohle. Die Reduktion ist auch moeglich durch Metalle wie Eisen, Zinn oder Zink in Gegenwart einer Saeure, mit Salzen wie Eisen-II-sulfat, Zinn-II-chlorid, Natriumsulfid,Natriumhydrogensulfid oder Natriumdithionit, oder mit Hydrazin in Gegenwart von Raney-Nickel .
Die Reaktionen werden bei 0 bis 250°C, vorzugsweise bei Raumtemperatur durchgefuehrt.

Die Cyclisierung zu Verbindungen der allgemeinen Formel I erfolgt bereits unter den Bedingungen der oben erwaehnten Reduktion der Nitrogruppe in Verbindungen der allgemeinen Formel IV. Gewuenschtenfalls kann diese Cyclisierung vervollstaendigt werden, indem man das Reaktionsprodukt aus der Reduktion der Nitrogruppe in einem Loesungsmittel oder Loesungsmittelgemisch wie Ethanol, Isopropanol, Eisessig, Benzol, Chlorbenzol, Glycol, Diethylenglycoldimethylether, Sulfolan oder Dimethylformamid auf Temperaturen zwischen 0° und 250°C, vorzugsweise auf die Siedetemperatur des Loesungsmittels erhitzt, gegebenenfalls in Gegenwart eines Kondensationsmittels wie Phosphoroxychlorid, Thionylchlorid, p-Toluolsulfonsaeure, Salzsaeure, Schwefelsaeure, Phosphorsaeure, Polyphosphorsaeure oder gegebenenfalls auch in Gegenwart einer Base wie Natriumhydroxyd, Kaliummethylat oder Kalium-tert.-butylat durchfuehrt. Die Cyclisierung kann jedoch auch ohne Loesungsmittel und/oder Kondensationsmittel durchgefuehrt werden.

Schema 2

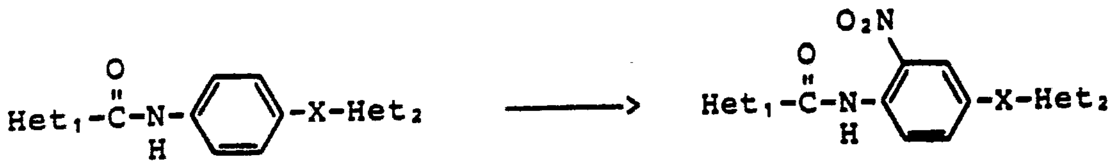

(V) (IV)

Die oben genannten Verbindungen der allgemeinen Formel IV lassen sich, wie aus Schema 2 ersichtlich, auch durch Nitrierung von Verbindungen der allgemeinen Formel V, in der $Het_1$, X und $Het_2$ die oben genannten Bedeutungen besitzen, herstellen.

Diese Nitrierungen werden in waessriger oder konzentrierter Schwefelsaeure, in Eisessig oder Acetanhydrid, gewuenschtenfalls in Gegenwart von Harnstoff, mittels Nitriersaeure oder waessriger oder konzentrierter Salpetersaeure bei Temperaturen zwischen -30°C und +80°C durchgefuehrt.

Schema 3

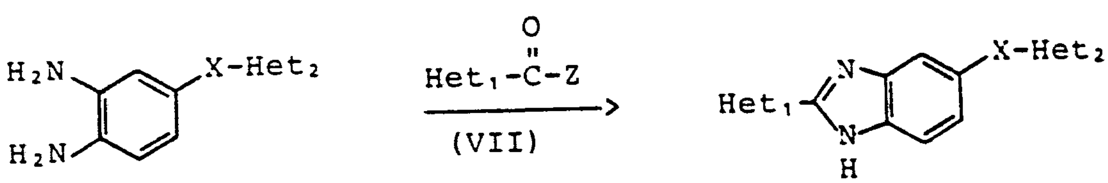

(VII)

(VI) (I)

Wie aus Schema 3 ersichtlich, lassen sich Verbindungen der allgemeinen Formel I auch herstellen, indem man Verbindungen der allgemeinen Formel VI, in der X und $Het_2$ die oben genannten Bedeutungen besitzen,

mit Verbindungen der allgemeinen Formel VII, in der $Het_1$ die oben genannten Bedeutungen besitzt und Z Wasserstoff, die Hydroxygruppe oder eine leicht abspaltbare Gruppe darstellt, umsetzt und zu Verbindungen der allgemeinen Formel I cyclisiert.

Insbesondere versteht man unter den Verbindungen der allgemeinen Formel VII Aldehyde, sowie Saeurehalogenide wie Saeurechloride, Carbonsaeureester wie Methyl- und Ethylester und andere aktivierte Carbonsaeurederivate, wie z.B. Anhydride, sowie die Carbonsaeuren selbst.

Ist die Verbindung der Formel VII ein Aldehyd findet die Umsetzung zur Schiffschen Base mit Verbindungen der allgemeinen Formel VI vorzugsweise in alkoholischem Medium statt, die anschließende Cyclisierung und Oxidation zu den Verbindungen der allgemeinen Formel I erfolgt durch Erwaermen des Reaktionsansatzes zum Rueckfluß in Gegenwart von Luftsauerstoff und katalytischen Mengen Saeure, wie z.B. Toluolsulfonsaeure.

Ist die Verbindung der allgemeinen Formel VII eine Carbonsaeure, so findet die Umsetzung mit Verbindungen der allgemeinen Formel VI zum Amid in Gegenwart eines wasserentziehenden Mittels, vorzugsweise in Polyphosphorsaeure bei Temperaturen zwischen 50 und 250°C, vorzugsweise zwischen 100 und 200°C statt.

Ist die Verbindung der allgemeinen Formel VII ein Carbonsaeurederivat, findet die Umsetzung mit Verbindungen der allgemeinen Formel VI zum Amid in inerten Loesungsmitteln, vorzugsweise in Methylenchlorid oder Pyridin statt und die anschließende Cyclisierung zu Verbindungen der allgemeinen Formel I wird in einem Loesungsmittel oder Loesungsmittelgemisch wie Ethanol, Isopropanol, Eisessig, Benzol,

Chlorbenzol, Glycol, Diethylenglycoldimethylether, Sulfolan oder Dimethylformamid bei Temperaturen zwischen 0° und 250°C, vorzugsweise jedoch bei der Siedetemperatur des Reaktionsgemisches, gegebenenfalls in Gegenwart eines Kondensationsmittels wie Phosphoroxychlorid, Thionylchlorid, p-Toluolsulfonsaeure, Salzsaeure, Schwefelsaeure, Phosphorsaeure, Polyphosphorsaeure oder gegebenenfalls auch in Gegenwart einer Base wie Natriumhydroxyd, Kaliummethylat oder Kalium-tert.-butylat durchgefuehrt. Die Cyclisierung kann jedoch auch ohne Loesungsmittel und/oder Kondensationsmittel durchgefueht werden.

Verbindungen der allgemeinen Formel I koennen auch nachtraeglich in eine andere Verbindung der Formel I umgewandelt werden.
Dies trifft zum Beispiel zu

fuer die Oxidation eines Fuenf- oder Sechsringes mit einem oder mehreren Stickstoffatomen zu den entsprechenden N-Oxiden. Die Oxidation wird zweckmaeßig mit einem oder mehreren Aequivalenten des verwendeten Oxidationsmittels durchgefuehrt, z.B. mit Wasserstoffperoxid in Eisessig, Trifluoressigsaeure oder in Ameisensaeure bei 20-100°C oder in Aceton bei 0-60°C, mit einer Persaeure wie Perameisensaeure oder m-Chlorperbenzoesaeure in Eisessig, Trifluoressigsaeure, Methylenchlorid oder Chloroform bei Temperaturen zwischen 0 und 60°C.

Ferner koennen die erhaltenen Verbindungen der allgemeinen Formel I anschließend gewuenschtenfalls in ihre physiologisch vertraeglichen Saeureadditionssalze mit anorganischen oder organischen Saeuren ueberfuehrt werden. Als Saeuren kommen hierfuer beispielsweise Salzsaeure, Bromwasserstoffsaeure, Schwefelsaeure, Phosphorsaeure, Fumarsaeure, Bernsteinsaeure, Weinsaeure, Zitronensaeure, Milchsaeure, Maleinsaeure oder Methansulfonsaeure in Betracht.

Wie bereits eingangs erwaehnt weisen die neuen Verbindungen der allgemeinen Formel I, deren Tautomere und deren physiologisch vertraegliche Saeureadditionssalze bei einer langen Wirkungsdauer ueberlegene pharmakologische Eigenschaften auf, insbesondere eine blutdrucksenkende und/oder positivinotrope Wirkung und/oder beeinflussen die Thrombozytenfunktion und verbessern die Mikrozirkulation.

Zur Herstellung von Arzneimitteln werden die Substanzen der allgemeinen Formel I in an sich bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die erfindungsgemaeßen neuen Substanzen der allgemeinen Formel I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt.

Derartige Zusaetze sind z.B. Tartrat- und Citratpuffer, Ethanol, Komplexbildner (wie Ethylendiamintetraessigsaeure und deren nicht toxische Salze) und hochmolekulare Polymere (wie fluessiges Polyethylenoxid) zur Viskositaetsregulierung. Feste Traegerstoffe sind z.B. Staerke, Lactose, Mannit, Methylcellulose, Talkum, hochdisperse Kieselsaeuren, hochmolekulare Fettsaeuren (wie Stearinsaeure), Gelatine, Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks- und Sueßstoffe enthalten.

Die erfindungsgemaeßen Verbindungen werden ueblicherweise in Mengen von 10-500 mg pro Tag bezogen auf 75 kg Koerpergewicht appliziert. Bevorzugt ist es, 2-3 mal pro Tag 1-2 Tabletten mit einem Wirkstoffgehalt von 5-200 mg zu verabreichen. Die Tabletten koennen retardiert sein, wodurch nur noch 1 mal pro Tag 1-2 Tabletten mit 10-500 mg Wirkstoff gegeben werden muß. Der Wirkstoff kann auch durch Injektion 1-8 mal pro Tag bzw. durch Dauerinfusion gegeben werden, wobei Mengen von 5-200 mg/Tag normalerweise ausreichen.

Bevorzugt im Sinne der vorliegenden Erfindung sind außer den in den Beispielen genannten Verbindungen die folgenden und deren Tautomere:

2-(3-Pyridinyl)-5-(3-oxo-2,3,4,5-tetrahydro-6-pyridazinyl)-benzimidazol

2-(2-Pyridinyl)-5-(3-oxo-2,3,4,5-tetrahydro-6-pyridazinyl)-benzimidazol

2-(4-Pyridazinyl)-5-(3-oxo-2,3,4,5-tetrahydro-6-pyridazinyl)benzimidazol

2-(5-Pyrimidinyl)-5-(3-oxo-2,3,4,5-tetrahydro-6-pyridazinyl)benzimidazol

2-(2-Pyrazinyl)-5-(3-oxo-2,3,4,5-tetrahydro-6-pyridazinyl)-benzimidazol

2-(1,2,4,5-Tetrazin-3-yl)-5-(3-oxo-2,3,4,5-tetrahydro-6-pyridazinyl)benzimidazol

2-(2-Thienyl)-5-(3-oxo-2,3,4,5-tetrahydro-6-pyridazinyl)-benzimidazol

2-(3-Thienyl)-5-(3-oxo-2,3,4,5-tetrahydro-6-pyridazinyl)-benzimidazol

2-(2-Pyrrolyl)-5-(3-oxo-2,3,4,5-tetrahydro-6-pyridazinyl)-benzimidazol

2-(2-Furyl)-5-(3-oxo-2,3,4,5-tetrahydro-6-pyridazinyl)-benzimidazol

2-(4-Imidazolyl)-5-(3-oxo-2,3,4,5-tetrahydro-6-pyridazinyl)-benzimidazol

2-(4-Thiazolyl)-5-(3-oxo-2,3,4,5-tetrahydro-6-pyridazinyl)-benzimidazol

2-(5-Oxazolyl)-5-(3-oxo-2,3,4,5-tetrahydro-6-pyridazinyl)-benzimidazol

2-(4-Pyrazolyl)-5-(3-oxo-2,3,4,5-tetrahydro-6-pyridazinyl)-benzimidazol

2-(1,2,4-Triazol-3-yl)-5-(3-oxo-2,3,4,5-tetrahydro-6-pyridazinyl)benzimidazol

2-(1,2,3-Thiadiazol-4-yl)-5-(3-oxo-2,3,4,5-tetrahydro-6-pyridazinyl)benzimidazol

2-(1,2,3-Thiadiazol-5-yl)-5-(3-oxo-2,3,4,5-tetrahydro-6-pyridazinyl)benzimidazol

2-(1,2,5-Thiadiazol-3-yl)-5-(3-oxo-2,3,4,5-tetrahydro-6-pyridazinyl)benzimidazol

2-(1,2,5-Oxadiazol-3-yl)-5-(3-oxo-2,3,4,5-tetrahydro-6-pyridazinyl)benzimidazol
2-(N-Oxy-3-pyridinyl)-5-(3-oxo-2,3,4,5-tetrahydro-6-pyridazinyl)benzimidazol
2-(N-Oxy-4-pyridinyl)-5-(3-oxo-2,3,4,5-tetrahydro-6-pyridazinyl)benzimidazol
2-(2-Chlor-4-pyridinyl)-5-(3-oxo-2,3,4,5-tetrahydro-6-pyridazinyl)benzimidazol
2-(2-Chlor-3-pyridinyl)-5-(3-oxo-2,3,4,5-tetrahydro-6-pyridazinyl)benzimidazol
2-(2-Hydroxy-4-pyridinyl)-5-(3-oxo-2,3,4,5-tetrahydro-6-pyridazinyl)benzimidazol
2-(3-Hydroxy-4-pyridinyl)-5-(3-oxo-2,3,4,5-tetrahydro-6-pyridazinyl)benzimidazol
2-(2,6-Dihydroxy-4-pyridinyl)-5-(3-oxo-2,3,4,5-tetrahydro-6-pyridazinyl)benzimidazol
2-(2-Cyano-4-pyridinyl)-5-(3-oxo-2,3,4,5-tetrahydro-6-pyridazinyl)benzimidazol
2-(2-Methyl-4-pyridinyl)-5-(3-oxo-2,3,4,5-tetrahydro-6-pyridazinyl)benzimidazol
2-(2-Methoxy-6-cyano-3-pyridinyl)-5-(3-oxo-2,3,4,5-tetrahydro-6-pyridazinyl)benzimidazol
2-(2-Methyl-5-pyrimidinyl)-5-(3-oxo-2,3,4,5-tetrahydro-6-pyridazinyl)benzimidazol
2-(2-Hydroxy-5-pyrimidinyl)-5-(3-oxo-2,3,4,5-tetrahydro-6-pyridazinyl)benzimidazol
2-(1,2-Dihydro-1-methyl-2-oxo-5-pyrimidinyl)-5-(3-oxo-2,3,4,5-tetrahydro-6-pyridazinyl)benzimidazol
2-(6-Methyl-4-pyrimidinyl)-5-(3-oxo-2,3,4,5-tetrahydro-6-pyridazinyl)benzimidazol
2-(3-Hydroxy-6-pyridazinyl)-5-(3-oxo-2,3,4,5-tetrahydro-6-pyridazinyl)benzimidazol
2-(5,6-Dimethyl-1,2,4-triazin-3-yl)-5-(3-oxo-2,3,4,5-tetrahydro-6-pyridazinyl)benzimidazol

2-(3-Methyl-5-pyrazolyl)-5-(3-oxo-2,3,4,5-tetrahydro-6-pyridazinyl)benzimidazol

2-(2-Methyl-4-oxazolyl)-5-(3-oxo-2,3,4,5-tetrahydro-6-pyridazinyl)benzimidazol

2-(2,6-Dihydroxy-4-pyrimidinyl)-5-(3-oxo-2,3,4,5-tetrahydro-6-pyridazinyl)benzimidazol

2-(N-Oxy-4-pyridinyl)-5-(5-methyl-3-oxo-2,3,4,5-tetrahydro-6-pyridazinyl)benzimidazol

2-(3-Hydroxy-4-pyridinyl)-5-(5-methyl-3-oxo-2,3,4,5-tetrahydro-6-pyridazinyl)benzimidazol

2-(2-Methyl-4-pyridinyl)-5-(5-methyl-3-oxo-2,3,4,5-tetrahydro-6-pyridazinyl)benzimidazol

2-(2-Chlor-3-pyridinyl)-5-(5-methyl-3-oxo-2,3,4,5-tetrahydro-6-pyridazinyl)benzimidazol

2-(2,6-Dihydroxy-4-pyridinyl)-5-(5-methyl-3-oxo-2,3,4,5-tetrahydro-6-pyridazinyl)benzimidazol

2-(2-Methoxy-6-cyano-3-pyridinyl)-5-(5-methyl-3-oxo-2,3,4,5-tetrahydro-6-pyridazinyl)benzimidazol

2-(1,2,3-Thiadiazol-5-yl)-5-(5-methyl-3-oxo-2,3,4,5-tetrahydro-6-pyridazinyl)benzimidazol

2-(1,2,3,-Thiadiazol-4-yl)-5-(5-methyl-3-oxo-2,3,4,5-tetrahydro-6-pyridazinyl)benzimidazol

2-(1,2,5- Thiadiazol -3-yl)-5-(5-hydroxymethyl-3-oxo-2,3,4,5-tetrahydro-6-pyridazinyl)benzimidazol

2-(1,2,5-Thiadiazol-3-yl)-5-(3-oxo-2,3,4,5-tetrahydro-1,2,4-triazin-6-yl)benzimidazol

2-(1,2,5-Thiadiazol-3-yl)-5-(2-oxo-2,3-dihydro-6H-1,3,4-oxadiazin-5-yl)benzimidazol

2-(1,2,5-Thiadiazol-3-yl)-5-(6-oxo-1,4,5,6-tetrahydro-1,2,4-triazin-3-yl)benzimidazol

2-(1,2,5-Thiadiazol-3-yl)-5-(5-oxo-4,5-dihydro-6H-1,3,4-oxadiazin-2-yl)benzimidazol

2-(1,2,5-Thiadiazol-3-yl)-5-(2-oxo-1,2-dihydro-5-pyrimidinyl)-benzimidazol

2-(1,2,5-Thiadiazol-3-yl)-5-(2-oxo-1,2-dihydro-5-pyrazinyl)-benzimidazol
2-(1,2,5-Thiadiazol-3-yl)-5-(2-oxo-2,3-dihydro-6H-1,3,4-thiadiazin-5-yl)benzimidazol
2-(1,2,5-Thiadiazol-3-yl)-5-(5-oxo-4,5-dihydro-6H-1,3,4-thiadiazin-2-yl)benzimidazol
2-(1,2,5-Thiadiazol-3-yl)-5-(3-cyan-6-methyl-2-oxo-1,2-dihydro-5-pyridinyl)benzimidazol
2-(1,2,5-Thiadiazol-3-yl)-5-(3-oxo-2,3-dihydro-6-pyridazinyl)-benzimidazol

**Beispiel 1**

2-(4-Pyridinyl)-5-(5-methyl-3-oxo-2,3,4,5-tetrahydro-6-pyridazinyl)benzimidazol

a) 19.6 g (0.1 mol) 6-(4-Aminophenyl)-5-methyl-3-oxo-2,3,4,5-tetrahydro-pyridazin wurde in Methylenchlorid suspendiert. Man gab 30.5 ml (0.22 mol) Triethylamin und anschließend unter Eiskuehlung und Feuchtigkeitsausschluß 21.3 g (0.12 mol) Isonicotinsaeurechlorid-Hydrochlorid portionsweise zu. Dabei ging das Ausgangsmaterial in Loesung. Nach 30 Minuten bei Raumtemperatur fiel 6-(4-(4-Pyridinylcarbonylamino)phenyl)-5-methyl-3-oxo-2,3,4,5-tetrahydro-pyridazin als Rohprodukt aus. Man versetzte mit Eiswasser und saugte das Kristallisat ab. Das Rohprodukt mit dem Schmelzintervall 275-285°C wurde ohne weitere Reinigung eingesetzt.
Ausbeute: 30.3 g

b) 15.4 g (0.05 mol) des obigen Rohprodukts in 100 ml konzentrierter Schwefelsaeure nitrierte man bei -15°C mit einer Mischung aus 2.3 ml rauchender Salpetersaeure und 2.3 ml konzentrierter Schwefelsaeure. Die Loesung wurde auf Eis gegossen, mit festem Natriumhydroxid abgepuffert, bis die Substanz ausfiel, die man absaugte und mit Wasser wusch. Man erhielt 10.3 g 6-(3-Nitro-4-(4-Pyridinylcarbonylamino)phenyl)-5-methyl-3-oxo-2,3,4,5-tetrahydropyridazin als Rohprodukt, das ohne weitere Reinigung eingesetzt wurde.

c) 10.2 g des in b) erhaltenen Produkts in 1 Liter Methanol hydrierte man bei Raumtemperatur in Gegenwart von 1 g 10 % Palladium auf Kohle, bis 1.9 Liter Wasserstoff aufgenommen waren. Diese Loesung engte man auf etwa 300 ml ein, gab 10 ml konzentrierter Salzsaeure zu und erhitzte drei Stunden unter Rueckfluß. Der Ansatz wurde zur Trockene eingeengt, der Rueckstand mit Wasser digeriert, mit 2 N Ammoniakloesung alkalisch gemacht. Das Wasser wurde vom ausgeschiedenen Oel dekantiert und das Oel (7.7 g) ueber eine Kieselgelsaeule gereinigt. Man verwendete 1 Liter Kieselgel und als Laufmittel eine Mischung aus Methylenchlorid und ammoniakalischem Methanol im Verhaeltnis 15:1 bis 10:1. Fraktionen der reinen Substanz wurde im Vakuum eingeengt, mit Ethanol digeriert und abgesaugt. Man erhielt 2.7 g der Titelverbindung mit dem Schmelzpunkt 240-243°C.

Beispiel 2

2-(4-Pyridinyl)-5-(2-(3-oxo-2,3,4,5-tetrahydro-6-pyridazinyl)-ethyl)benzimidazol

a) 11.5 g 6-((4-Acetamido-phenyl)-2-ethyl)-3-oxo-2,3,4,5-tetrahydropyridazin in 100 ml konzentrierter Salzsaeure wurde bei 0°C mit einer Mischung aus 2 ml rauchender Salpetersaeure und 2 ml konzentrierter Schwefelsaeure nitriert. Man versetzte mit Eiswasser und saugte den kristallinen Niederschlag ab. Man erhielt 12.4 g Rohprodukt, die man mit einer Essigester-Ether-Mischung digerierte. Man saugte ab und erhielt 9.9 g Rohprodukt mit dem Schmelzintervall 135-160°C, das ohne Reinigung weiter umgesetzt wurde.

b) Das obige Rohprodukt wurde in 100 ml Ethanol und 10 ml konzentrierter Salzsaeure unter Rueckfluß zum Sieden erhitzt. Nach einer Stunde engte man im Vakuum ein, suspendierte den Rueckstand in Wasser und extrahierte mit Methylenchlorid. Man trocknete die organische Phase, filtrierte und engte im Vakuum ein. Das zurueckbleibende Oel wurde mit Ether angerieben und die erhaltenen Kristalle abgesaugt. Die so erhaltenen 3.6 g 6-((3-Nitro-4-amino-phenyl)-2-ethyl)-3-oxo-2,3,4,5-tetrahydro-pyridazin mit dem Schmelzbereich 175-190°C wurden ohne weitere Reinigung eingesetzt.

c) 3.5 g des in b) erhaltenen Produktes hydrierte man in 100 ml Methanol in Gegenwart von 0.3 g 10 % Palladium auf Kohle bei Normaldruck und Raumtemperatur. Man saugte den Katalysator ab und engte die Loesung im Vakuum zur Trockene ein. Den Rueckstand (3.1 g) suspendierte man in Methylenchlorid, gab 2.8 g Triethylamin und anschließend portionsweise unter Eiskuehlung 3.6 g Isonicotinsaeurechlorid-Hydrochlorid zu. Anschließend gab man 10 ml Pyridin zu und ruehrte bei Raumtemperatur. Man versetzte mit Wasser, trennte die organische Phase ab, extrahierte die waessrige Phase zweimal mit Methylenchlorid, dann die vereinigten organischen Phaseneinmal mit1 N Salzsaeure. Die organische Phase wurde im Vakuum eingeengt, der Rueckstand in 100 ml Ethanol und 20 ml konzentrierter Salzsaeure aufgenommen und einen Tag lang unter Rueckfluß zum Sieden erhitzt. Das Loesungsmittel wurde im Vakuum entfernt und der Rueckstand saeulenchromatographisch an 500 ml Kieselgel mit einer Mischung aus Methylenchlorid und methanolischem Ammoniak im Verhaeltnis 30:1 gereinigt. Die entsprechenden Fraktionen wurden im Vakuum eingeengt. Man erhielt so 200 mg der Titelverbindung mit dem Schmelzpunkt 130-135°C.

Beispiel 3

2-(1,2,5-Thiadiazol-3-yl)-5-(5-methyl-3-oxo-2,3,4,5-tetrahydro-6-pyridazinyl)benzimidazol

1.86 g 6-(4-Amino-3-nitro-phenyl)-5-methyl-3-oxo-2,3,4,5-tetrahydropyridazin hydrierte man in Gegenwart von 0.3 g 10 % Palladium auf Kohle in 50 ml Methanol bei Normaldruck und Raumtemperatur. Man engte die Loesung nach Filtration im Vakuum ein, gab 35 g Polyphosphorsaeure zu, trug unter Ruehren 10 g Phosphorpentoxid und dann 2 g 1,2,5-Thiadiazol-3-carbonsaeure ein und erhitzte den Ansatz unter starkem Ruehren fuenf Stunden auf 150°C. Man ließ auf 70°C abkuehlen und zersetzte den Ansatz vorsichtig mit Eis und kaltem Wasser (60 ml). Dann goß man auf 500 ml Wasser, saugte den Niederschlag ab und behandelte den Niederschlag mit 5 ml konzentrierter waessriger Ammoniakloesung. Nach Stehen ueber Nacht saugte man den Niederschlag ab und kristallisierte aus Ethanol.
Man erhielt die Titelverbindung in 21 % Ausbeute mit dem Schmelzpunkt 260° C.

**Beispiel 4**

Analog Beispiel 3 wurden erhalten:

| | Bezeichnung | Ausbeute [%] | Reinigung Schmp. (°C) Loesungsmittel |
|---|---|---|---|
| a) | 2-(3-Pyridinyl)-5-(5-methyl-3-oxo-2,3,4,5-tetrahydro-6-pyridazinyl)benzimidazol | 10 | Umkrist. aus Ether 158-160°C |
| b) | 2-(3-Hydroxy-4-pyridinyl)-5-(5-methyl-3-oxo-2,3,4,5-tetrahydro-6-pyridazinyl)-benzimidazol | | |
| c) | 2-(2-Pyrazinyl)-5-(5-methyl-3-oxo-2,3,4,5-tetrahydro-6-pyridazinyl)benzimidazol | | |
| d) | 2-(5-Pyrimidinyl)-5-(5-methyl-3-oxo-2,3-dihydro-6-pyridazinyl)benzimidazol | 3 | Umkristallisation aus Ether 188-190°C |
| e) | 2-(4-Pyridazinyl)-5-(5-methyl-3-oxo-2,3,4,5-tetrahydro-6-pyridazinyl)benzimidazol | 8 | Umkristallisation aus Ethanol 293-295°C |
| f) | 2-(2-Furyl)-5-(5-methyl-3-oxo-2,3,4,5-tetrahydro-6-pyridazinyl)-benzimidazol | 11 | Kristallisation aus Ethanol/$H_2O$ 1:1 262-266°C |
| g) | 2-(1,2,3-Thiadiazol-4-yl)-5-(5-methyl-3-oxo-2,3,4,5-tetrahydro-6-pyridazinyl)benzimidazol | 15 | Umkristallisation aus Ethanol 250-252°C (Zers.) |

| | Bezeichnung | Ausbeute [%] | Reinigung Schmp. (°C) Loesungsmittel |
|---|---|---|---|
| h) | 2-(2-Thienyl)-5-(5-methyl-3-oxo-2,3,4,5-tetrahydro-6-pyridazinyl)-benzimidazol | 43 | 156-160 (Zers.) |
| i) | 2-(2-Pyrrolyl)-5-(5-methyl-3-oxo-2,3,4,5-tetrahydro-6-pyridazinyl)-benzimidazol | 3 | Umkristallisation aus Ethanol >300°C |
| j) | 2-(4-Imidazolyl)-5-(5-methyl-3-oxo-2,3,4,5-tetrahydro-6-pyridazinyl)benzimidazol | 4 | Umkristallisation aus Isopropanol >300°C |
| k) | 2-(5-Oxazolyl)-5-(5-methyl-3-oxo-2,3,4,5-tetrahydro-6-pyridazinyl)-benzimidazol | | |
| l) | 2-(1,2,4-Triazol-3-yl)-5-(5-methyl-3-oxo-2,3,4,5-tetrahydro-6-pyridazinyl)benzimidazol | | |
| m) | 2-(5-Isoxazolyl)-5-(5-methyl-3-oxo-2,3,4,5-tetrahydro-6-pyridazinyl)benzimidazol | 17 | Kieselgelsaeule $CH_2Cl_2$/MeOH = 98 : 2 254-256°C (Zers) (Methanol) |
| n) | 2-(2-Methyl-4-pyridinyl)-5-(5-methyl-3-oxo-2,3,4,5-tetrahydro-6-pyridazinyl)benzimidazol | 45 | Kieselgelsäule $CH_2Cl_2$/methanol. $NH_3$ = 15:1 243-246°C |

**Beispiel 5**

Analog dem Beispiel 3 erhielt man aus den entsprechenden Amino-nitroverbindungen durch Reduktion und nachfolgende Umsetzung mit Isonicotinsaeure die folgenden Verbindungen:

| | Bezeichnung | Ausbeute [%] | Reinigung Schmp. (°C) Loesungsmittel |
|---|---|---|---|
| a) | 2-(4-Pyridinyl)-5-(3-cyan-6-methyl-2-oxo-1,2-dihydro-5-pyridyl)benzimidazol | | |
| b) | 2-(4-Pyridinyl)-5-(3-oxo-2,3,4,5-tetrahydro-6-pyridazinyl)-benzimidazol | 25 | Kieselgelsaeule $CH_2Cl_2$/MeOH = 15 : 1 316-318° C (Ethanol) |
| c) | 2-(4-Pyridinyl)-5-(3-oxo-2,3-dihydro-6-pyridazinyl)benzimidazol | 61 | Kristallisiert aus Ethanol >320°C |
| d) | 2-(4-Pyridinyl)-5-(2-oxo-1,2-dihydro-5-pyrimidinyl)benzimidazol | | |
| e) | 2-(4-Pyridinyl)-5-(2-oxo-1,2-dihydro-5-pyrazinyl)-benzimidazol | 22 | Umkristallisation aus Methanol >300°C |
| f) | 2-(4-Pyridinyl)-5-(6-oxo-1,4,5,-6-tetrahydro-1,2,4-triazin-3-yl)-benzimidazol | | |

| | Bezeichnung | Ausbeute [%] | Reinigung, Schmp. (°C) Loesungsmittel |
|---|---|---|---|
| g) | 2-(4-Pyridinyl)-5-(3-oxo-2,3,4,5-tetrahydro-1,2,4-triazin-6-yl)-benzimidazol | 5 | Kieselgelsäule $CH_2Cl_2/CH_3OH$(10:1) >300°C (Methanol) |
| h) | 2-(4-Pyridinyl)-5-(2-oxo-2,3-dihydro-6H-1,3,4-oxadiazin-5-yl)-benzimidazol x 0.5 $CH_3COOH$ | 35 | Kristallisiert aus Eisessig 306-310° |
| i) | 2-(4-Pyridinyl)-5-(5-oxo-4,5-dihydro-6H-1,3,4-oxadiazin-2-yl)-benzimidazol | 3 | Kieselgelsäule $CH_2Cl_2/CH_3OH$(10:1) >300°C (Ethanol) |
| j) | 2-(4-Pyridinyl)-5-(2-oxo-2,3-dihydro-6H-1,3,4-thiadiazin-5-yl)-benzimidazol | | |
| k) | 2-(4-Pyridinyl)-5-(5-oxo-4,5-dihydro-6H-1,3,4-thiadiazin-2-yl)-benzimidazol | | |
| 1) | 2-(4-Pyridinyl) -5-(2-oxo-1,2-dihydro-4-methyl-pyrimidinyl) benzimidazol | 30 | Kieselgelsäule $CH_2Cl_2/CH_3OH$ (&:1) > 300°C (Methanol) |

Die nachstehenden Beispiele betreffen pharmazeutische Zubereitungen, die Verbindungen der Formel I oder ihre Säureadditionssalze enthalten. Als Wirkstoff ist beispielhaft die Verbindung des Beispiels 1 eingesetzt.

A. Tabletten

Ein Gemisch von 1 kg der Verbindung des Beispiels 1, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 10 mg Wirkstoff enthält.

B. Dragees

Analog A werden Tabletten gepreßt, die anschließend in üblicher Weise mit einem Überzug aus Saccharose, Kartoffelstärke, Talk, Tragant und Farbstoff überzogen werden.

C. Kapseln

1 kg der Verbindung des Beispiels 1 wird in üblicher Weise in Hartgelatinekapseln gefüllt, so daß jede Kapsel 5 mg Wirkstoff enthält.

D. Ampullen

Eine Lösung von 1 kg der Verbindung des Beispiels 1 in 100 l zweifach destilliertem Wasser wird steril filtriert, in Ampullen abgefüllt, unter sterilen Bedingungen lyophilisiert und steril verschlossen. Jede Ampulle enthält 2 mg Wirkstoff.

## Patentansprueche

1. Heterocyclisch substituierte Benzimidazole der allgemeinen Formel I

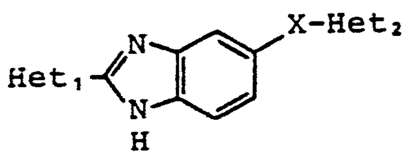

(I)

in welcher

$Het_1$ einen heterocyclischen Fuenfring mit 1-4 Heteroatomen oder einen heterocyclischen Sechsring mit 1-5 Heteroatomen darstellt, wobei die Heteroatome der vorgenannten Fuenf- und Sechsringe gleich oder verschieden sein koennen und Stickstoff, Sauerstoff oder Schwefel bedeuten und gegebenenfalls an einem oder mehreren Stickstoffatomen ein Sauerstoffatom tragen koennen, und die vorgenannten Fuenf- und Sechsringe gegebenenfalls durch eine oder mehrere Alkyl-, Alkoxy-, Alkylmercapto-, Hydroxy-, Nitro-, Amino-, Halogen- oder Cyangruppen substituiert sein koennen,

$Het_2$ einen heterocyclischen Sechsring mit 1-5 Heteroatomen darstellt, wobei die Heteroatome gleich oder verschieden sein koennen und Stickstoff, Sauerstoff oder Schwefel bedeuten,

und die Sechsringe gewuenschtenfalls durch eine oder mehrere Alkyl-, Alkoxy-, Alkoxyalkyl-, Alkylmercapto-, Hydroxy-, Hydroxyalkyl-, Amino-, Halogen-, oder Cyangruppen substituiert sein koennen,

X eine Bindung, eine $C_1$-$C_4$-Alkylengruppe oder die Vinylengruppe bedeutet,

deren Tautomere und deren physiologisch vertraegliche Salze anorganischer und organischer Saeuren.

2. Verbindungen gemaeß Anspruch 1, dadurch gekennzeichnet, daß

$Het_1$ den Pyrrol-, Furan-, Thiophen-, Pyrazol-, Imidazol-, Isoxazol-, Thiazol-, Oxazol-, Triazol-, Tetrazol-, Thiadiazol-, Oxadiazol-, Pyridin-, N-Oxy-pyridin-, Pyrazin-, N,N'-Dioxy-pyrazin-, Pyrimidin-, N,N'-Dioxypyrimidin-, Pyridazin-, Oxazin-, Thiazin-, Triazin- oder Tetrazinrest darstellt, sowie deren Methyl-, Ethyl-, Methoxy-, Hydroxy-, Ethoxy-, Methylmercapto-, Ethylmercapto- und chlorsubstituierten Derivate,

$Het_2$ den 3-Oxo-2,3,4,5-tetrahydro-6-pyridazinyl-, 5-Methyl-3-oxo-2,3,4,5-tetrahydro-6-pyridazinyl-, 5-Hydroxymethyl-3-oxo-2,3,4,5-tetrahydro-6-pyridazinyl-, 3-Cyan-6-methyl-2-oxo-1,2-dihydro-5-pyridinyl-, 3-Oxo-2,3,4,5-tetrahydro-1,2,4-triazin-6-yl, 2-Oxo-4,5-dihydro-6H-1,3,4-oxadiazin-5-yl-, 6-Oxo-1,4,5,6-tetrahydro-1,2,4-triazin-3-yl-,

5-Oxo-4,5-dihydro-6H-1,3,4-oxadiazin-2-yl-, 2-Oxo-1,2-dihydro-5-pyrimidinyl-, 2-Oxo-1,2-dihydro-5-pyrazinyl-,

5-Oxo-4,5-dihydro-6H-1,3,4-thiadiazin-2-yl-, oder den 5-Oxo-4,5-dihydro-6H,1,3,4-thiadiazin-5-yl-rest und

X einen Valenzstrich, die Vinylengruppe oder die Ethylengruppe

bedeuten.

3. Verfahren zur Herstellung von Verbindungen der allgemeinen Formel I

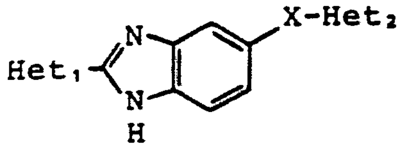

(I)

in welcher

$Het_1$ einen heterocyclischen Fuenfring mit 1-4 Heteroatomen oder einen heterocyclischen Sechsring mit 1-5 Heteroatomen darstellt, wobei die Heteroatome der vorgenannten Fuenf- und Sechsringe gleich oder verschieden sein koennen und Stickstoff, Sauerstoff oder Schwefel bedeuten und gegebenenfalls an einem oder mehreren Stickstoffatomen ein Sauerstoffatom tragen koennen, und die vorgenannten Fuenf- und Sechsringe gegebenenfalls durch eine oder mehrere Alkyl-, Alkoxy-, Alkylmercapto-, Hydroxy-, Nitro-, Amino-, Halogen- oder Cyangruppen substituiert sein koennen,

$Het_2$ einen heterocyclischen Sechsring mit 1-5 Heteroatomen darstellt, wobei die Heteroatome gleich oder verschieden sein koennen und Stickstoff, Sauerstoff oder Schwefel bedeuten,

und die Sechsringe gewuenschtenfalls durch eine oder mehrere Alkyl-, Alkoxy-, Alkoxyalkyl-, Alkylmercapto-, Hydroxy-, Hydroxyalkyl-, Amino-, Halogen-, oder Cyangruppen substituiert sein koennen,

X eine Bindung, eine $C_1$-$C_4$-Alkylengruppe oder die Vinylengruppe bedeutet,

deren Tautomere und deren physiologisch vertraeglichen Salzen anorganischer und organischer Saeuren, dadurch gekennzeichnet, daß man

a) eine Verbindung der allgemeinen Formel IV

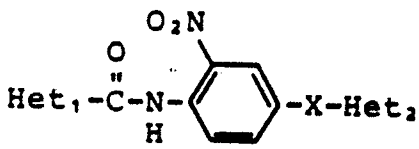

(IV),

in der

$Het_1$, $Het_2$ und X die in Anspruch 1 angegebenen Bedeutungen haben,

reduziert und cyclisiert

oder

b) eine Verbindung der allgemeinen Formel VI

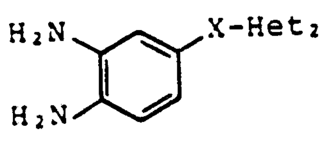

(VI),

in der

X und $Het_2$ die in Anspruch 1 angegebenen Bedeutungen haben

mit

einer Verbindung der allgemeinen Formel VII

$$Het_1-\overset{\overset{O}{\|}}{C}-Z \qquad (VII),$$

in der

$Het_1$ die in Anspruch 1 angegebene Bedeutung hat und
Z Wasserstoff, die Hydroxygruppe oder eine leicht abspaltbare Gruppe darstellt,

umsetzt und cyclisiert

und

anschließend gewuenschtenfalls erhaltene Verbindungen der allgemeinen Formel I in andere Verbindungen der allgemeinen Formel I umwandelt

sowie

gegebenenfalls die Verbindungen in physiologisch vertraegliche Salze anorganischer und organischer Saeuren ueberfuehrt.

4. Verfahren gemaeß Anspruch 3 zur Herstellung von Verbindungen der Formel I,

in der

Het$_1$ den Pyrrol-, Furan-, Thiophen-, Pyrazol-, Imidazol-, Isoxazol-, Thiazol-, Oxazol-, Triazol-, Tetrazol-, Thiadiazol-, Oxadiazol-, Pyridin-, N-Oxy-pyridin-, Pyrazin-, N,N'-Dioxy-pyrazin-, Pyrimidin-, N,N'-Dioxypyrimidin-, Pyridazin-, Oxazin-, Thiazin-, Triazin- oder Tetrazinrest darstellt, sowie deren Methyl-, Ethyl-, Methoxy-, Hydroxy-, Ethoxy-, Methylmercapto-, Ethylmercapto- und chlorsubstituierten Derivate,

Het$_2$ den 3-Oxo-2,3,4,5-tetrahydro-6-pyridazinyl-, 5-Methyl-3-oxo-2,3,4,5-tetrahydro-6-pyridazinyl-, 5-Hydroxymethyl-3-oxo-2,3,4,5-tetrahydro-6-pyridazinyl-, 3-Cyan-6-methyl-2-oxo-1,2-dihydro-5-pyridinyl-, 3-Oxo-2,3,4,5-tetrahydro-1,2,4-triazin-6-yl, 2-Oxo-4,5-dihydro-6H-1,3,4-oxadiazin-5-yl-, 6-Oxo-1,4,5,6-tetrahydro-1,2,4-triazin-3-yl-,

5-Oxo-4,5-dihydro-6H-1,3,4-oxadiazin-2-yl-, 2-Oxo-1,2-dihydro-5-pyrimidinyl-, 2-Oxo-1,2-dihydro-5-pyrazinyl-,

5-Oxo-4,5-dihydro-6H-1,3,4-thiadiazin-2-yl-, oder den 5-Oxo-4,5-dihydro-6H,1,3,4-thiadiazin-5-yl-rest und

X einen Valenzstrich, die Vinylengruppe oder die Ethylengruppe

bedeuten.

5. Verwendung von Verbindungen gemaeß Anspruch 1 und 2 zur Behandlung von Herz- und Kreislauferkrankungen.

6. Arzneimittel, enthaltend mindestens eine Verbindung gemaeß Anspruch 1 und 2 neben ueblichen Traeger- und Hilfsstoffen.

0240026

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung: EP 87104925.0

## EINSCHLÄGIGE DOKUMENTE

| Kategorie | Kennzeichnung des Dokuments mit Angabe, soweit erforderlich, der maßgeblichen Teile | Betrifft Anspruch |
|---|---|---|
| X | US - A - 3 336 192 (SARETT)<br>* Formel I; Beispiele 1-6 *<br>-- | 1 |
| P,X | EP - A1 - 0 196 005 (THOMAE)<br>* Formel I; Beispiele 1-7; Spalte 10, Zeilen 12 ff *<br>-- | 1,6 |
| P,X | EP - A2 - 0 193 013 (MERCK)<br>* Ansprüche 1,3,5-8; Formel I; Seite 18, Zeilen 10 ff *<br>-- | 1,3,6 |
| A | DE - A1 - 2 711 362 (HOECHST)<br>* Formel I; Ansprüche 2,3 *<br>-- | 1,3,6 |
| A | US - A - 4 329 459 (MCCALL)<br>* Beispiele 2-5,7,16 *<br>-- | 1,6 |

KLASSIFIKATION DER ANMELDUNG (Int. Cl 4)

C 07 D 401/04
C 07 D 401/06
C 07 D 401/14
C 07 D 403/04
C 07 D 403/06
C 07 D 403/14
C 07 D 405/04
C 07 D 405/06
C 07 D 405/14
C 07 D 409/04
C 07 D 409/06
C 07 D 409/14
C 07 D 413/04
C 07 D 413/06
C 07 D 413/14
C 07 D 417/04
C 07 D 417/06
C 07 D 417/14
A 61 K 31/41
A 61 K 31/50

RECHERCHIERTE SACHGEBIETE (Int. Cl 4)

C 07 D 401/00
C 07 D 403/00
C 07 D 405/00
C 07 D 409/00
C 07 D 413/00
C 07 D 417/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-4,6
Unvollständig recherchierte Patentansprüche: –
Nicht recherchierte Patentansprüche: 5
Grund für die Beschränkung der Recherche:

Artikel 52(4) EPÜ; Verfahren zur therapeutischen Behandlung des menschlichen oder tierischen Körpers

| Recherchenort | Abschlußdatum der Recherche | Prüfer |
|---|---|---|
| WIEN | 23-06-1987 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 06.82

0240026

Nummer der Anmeldung

Europäisches Patentamt

# EUROPÄISCHER TEILRECHERCHENBERICHT

EP 87104925.0

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl. 4) |
|---|---|---|---|
| A | US - A - 4 208 340 (LEE)<br>* Spalte 5, Zeilen 57-65 *<br>-- | 1 | |
| A | US - A - 3 325 506 (JONES)<br>* Spalte 5, Zeilen 10-30 *<br>-- | 1 | |
| A | FR - A - 1 519 964 (HOECHST)<br>* Anspruch 1 *<br>-- | 1,3 | |
| A | DE - A1 - 2 804 835 (HOECHST)<br>* Ansprüche 1-3; Formel I *<br>---- | 1,3,6 | RECHERCHIERTE SACHGEBIETE (Int Cl 4) |

EPA Form 1505.3 06.78